# EUROPEAN PATENT APPLICATION

(11) **EP 1 188 771 A1**
(43) Date of publication of application: **20.03.2002**
(21) Application number: 00203216.7
(22) Date of filing: 15.09.2000
(51) Int. Cl.: C07K 16/00, C12N 15/10

(54) **Libraries of human heavy chain variable fragments in a functional format**

(71) Applicant: U-BiSys B.V., 3584 CX Utrecht (NL)
(72) Inventor: Logtenberg, Ton, 3985 MK Werkhoven (NL); Houtzager, Erwin, 3958 XD Amorongen (NL)
(74) Representative: Prins, Adrianus Willem

(57) **Abstract**

Method for producing a human monoclonal antibody, said method comprising: Providing a library of binding molecules, the binding domain of which consists essentially of human heavy chain variable fragments in a functional format, selecting from said library at least one heavy chain variable fragment having a desired affinity, and inserting a nucleic acid encoding said heavy chain variable fragment into a nucleic acid encoding the complementary part of at least a heavy chain of said human monoclonal antibody, allowing for expression of the resulting heavy chain and for assembly of said heavy chain with a desired light chain, and producing a human monoclonal antibody. Said functional format is due to fusion to a phage-associated structural protein.

## Description

### Technical Field:

The present invention relates to the fields of molecular biology and immunology and in particular to the field of designing, for example, human antibodies having a desired binding affinity through display and selection techniques.

### Background:

The exposure to a highly diverse and continuously changing environment requires a dynamic immune system that is able to rapidly adapt in order to adequately respond to potentially harmful microorganisms. Higher organisms have evolved specialized molecular mechanisms to ensure the implementation of clonally-distributed, highly diverse repertoires of antigen-receptor molecules expressed by cells of the immune system: immunoglobulin (Ig) molecules on B lymphocytes and T cell receptors on T lymphocytes. For B lymphocytes, a primary repertoire of (generally low affinity) Ig receptors is established during B cell differentiation in the bone marrow as a result of rearrangement of germline-encoded gene segments. Further refinement of Ig receptor specificity and affinity takes place in peripheral lymphoid organs where antigen-stimulated B lymphocytes activate a somatic hypermutation machinery that specifically targets the immunoglobulin variable (V) regions. During this process, B cell clones with mutant Ig receptors of higher affinity for the inciting antigen are stimulated into clonal proliferation and maturation into antibody-secreting plasma cells (reviewed in 1).

In recent years, recombinant DNA technology has been used to mimic many aspects of the processes that govern the generation and selection of natural human antibody repertoires (reviewed in 2,3). For instance, the construction of large repertoires of antibody fragments expressed on the surface of filamentous phage particles and the selection of phages by "panning" on antigens has been developed as a versatile and rapid method to obtain antibodies of desired specificities (reviewed in 4,5). Further optimization of the affinity of individual phage antibodies has been achieved by creating mutant antibody repertoires that are expressed on bacteriophage particles and sampled for higher affinity mutants by selection for binding to antigen under stringent conditions (reviewed in 6). Various approaches have been used to create mutated antibody repertoires, including chain shuffling (7,8), error prone PCR (9), use of *E. coli mutator strains* (10) *or approaches more* specifically directed to the complementarity determining regions ("CDRs") of the antibody molecule, like CDR "walking" and parsimonious mutagenesis (11-13).

Libraries created so far have a more limited span of specificities than possible. This is in large part due to the fact that many specificities present are not expressed or exposed properly by the organism, for example, chosen for expression of the library components. This is most likely due to a lack of adaptation of the expression products to the expression environment.

Furthermore, the libraries created so far, if they contain a desired specificity, require engineering of the nucleic acid encoding the specificity in order to be able to create a fully human monoclonal antibody. For instance, in single chain Fv molecules, the light chain encoding sequence and the heavy chain encoding sequence are separated from the linker sequence and separately inserted into a complementary part of a heavy chain encoding sequence and a light chain encoding sequence. Upon this rearranging of the variable parts, specificity and affinity may change.

The present invention solves these problems at least in part. Other advantages and embodiments of the present invention will be clear from the detailed description below.

### Disclosure of the Invention

The invention now provides libraries which comprise binding molecules that are adapted to expression in the expression organism, but which are also transferable to a human context without undergoing a change in conformation and/or build up.

Thus, the present invention provides a method for producing a human monoclonal antibody, said method comprising: providing a library of binding molecules, the binding domain of which consists essentially of human heavy chain variable fragments in a functional format, selecting from said library at least one heavy chain variable fragment having a desired binding affinity, and inserting a nucleic acid encoding said heavy chain variable fragment into a nucleic acid encoding the complementary part of at least a heavy chain of said human monoclonal antibody, allowing for expression of the resulting heavy chain and for assembly of said heavy chain with a desired light chain, and producing a human monoclonal antibody. The present inventors have found that having only a heavy chain derived variable fragment determining the binding affinity of the binding molecules in the library, that, as long as they are presented in a functional format, this will suffice for creating a library at least as large as the known ones, but typically will allow for producing even larger libraries. Also, the loss of specificities and affinities because of expression problems can be reduced, especially according to the preferred embodiments as disclosed herein below. A heavy chain variable fragment is defined as anything based on a fragment the size of a CDR (complementarity determining region) of a heavy chain (e.g., CDR 3) to a heavy chain variable fragment as usually defined in the art. Also, the way the heavy chain variable fragments are encoded, allows for the direct insertion into a (preferably) standard complementary part of the heavy chain encoding nucleic acid without significantly altering its conformation, affinity and/or specificity. The resulting heavy chain (upon expression) can then be assembled with a (preferably standard) light chain. However, this light chain will typically not have any significant binding affinity for the molecule recognized by the heavy chain variable fragment.

The nucleic acids encoding the heavy and light chains of the resulting human monoclonal antibody may be the same or different. They typically are expressed in a eukaryotic cell, preferably a human cell, preferably a cell like PER.C6. It may be either transient expression or from insertions in the host cell's genome; the latter being preferred.

### Detailed Description of the Invention

In a preferred embodiment, the methods of the invention are carried out in a manner wherein the heavy chain variable fragment is in a functional format through fusion to a structural protein designed for that purpose. A functional format means that its conformation is such that it retains it binding affinity whether it is in phage display, or in its normal heavy chain environment. Methods of keeping heavy chain variable fragments in such a conformation are an important aspect of the present invention. It is disclosed herein how to provide amino acid sequences capable of simulating the conformation of the heavy chain variable fragment in phage display surroundings the way they are in the natural surroundings. One way is fusing a variable fragment with a known affinity to random sequences, expressing the resulting nucleic acids and selecting for the known affinity.

In another preferred embodiment, the equality of the conformation of the phage display fragment and the fragment in the heavy chain environment is removal of at least one sequence which is responsible for associating with a light chain. In this format, an indifferent light chain variable fragment can be used as a structural amino acid sequence. According to the invention, the heavy chain variable fragment is preferably inserted into a standard human heavy chain encoding nucleic acid, derived from a human antibody backbone which is prevalent in the population, these include, but are not limited to members of the VH1, VH3 or VH4 gene families.

The same is true for the light chain. These include, but are not limited to members of the Vkappa1, Vkappa3 and Vlambda3 gene familes.

This way, the invention provides a kit of parts consisting of heavy chain variable fragments having the desired binding affinity to cut from the library and a set of ready to use monoclonal antibody encoding nucleic acids to insert them in.

Thus, the invention also provides a human monoclonal antibody obtainable by a method according to the invention as disclosed above. As explained previously herein, the invention provides a method for producing a structural amino acid sequence or a nucleic acid sequence encoding such an amino acid sequence for keeping a human heavy chain variable fragment in a functional format upon expression of a nucleic acid encoding such a fragment in a fusion with a nucleic acid encoding a protein expressed associated with the surface of a phage particle, comprising fusing a nucleic acid sequence encoding a possible structural amino acid sequence to a nucleic acid which is a fusion of a human heavy chain variable fragment with a known binding affinity and the nucleic acid encoding a protein expressed associated with the surface of a phage particle and expressing said nucleic acid in the context of a suitable phage expression system and selecting fusions which expose the desired binding affinity. The fusions in functional alignment basically mean that the order in which the sequences are present can be different and be functional. The heavy chain variable fragment and the structural amino acid sequence encoding parts should be next to each other, in either direction. The phage surface protein encoding nucleic acid can be on either side. The linkage may be direct or indirect. The amino acid sequence designed for keeping a heavy chain variable fragment in the proper conformation will work for other heavy chain variable fragments as well. The invention thus also includes these amino acid sequences (proteinaceous substances) and their encoding nucleic acids. Thus, one can make a library of heavy chain variable fragments in proper conformation, because of the presence of the novel structural sequence.

The invention further comprises a method for making a library for use in a method according to the invention, comprising cloning a number of randomized nucleic acids derived from a heavy chain variable fragment in functional alignment with a nucleic acid encoding a proteinaceous substance as disclosed hereinabove, and providing the resulting nucleic acid in functional alignment with a nucleic acid encoding a protein expressed associated with the surface of a phage particle and expressing the resulting nucleic acids comprising said heavy chain variable fragment, the proteinaceous substance encoding acid and said surface protein encoding nucleic acid in the context of a suitable phage expression system, thus producing said library. The invention also provides a phage display library obtainable by a method disclosed above.

### Examples

### Example 1

Generation of a library of heavy chain variable regions using a soluble variable heavy chain 3 domain (sVH3).

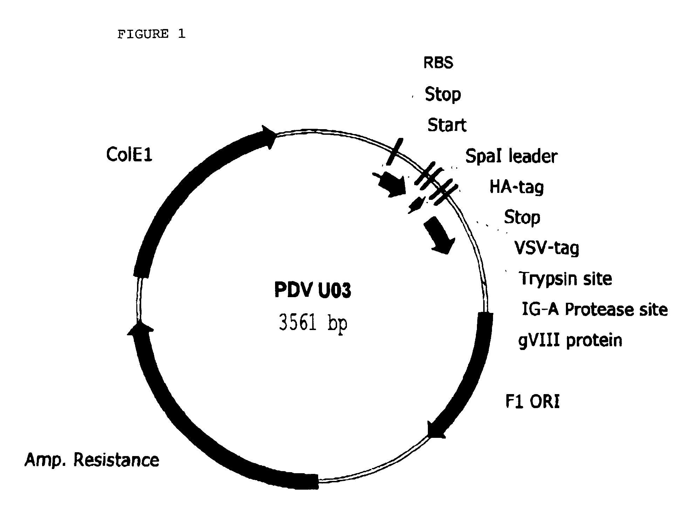

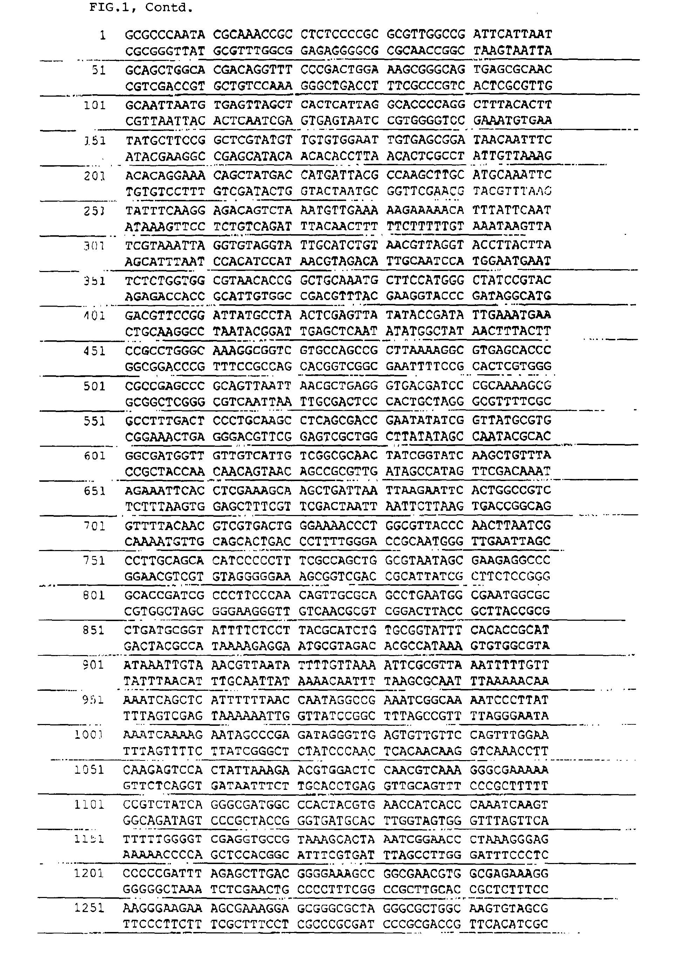

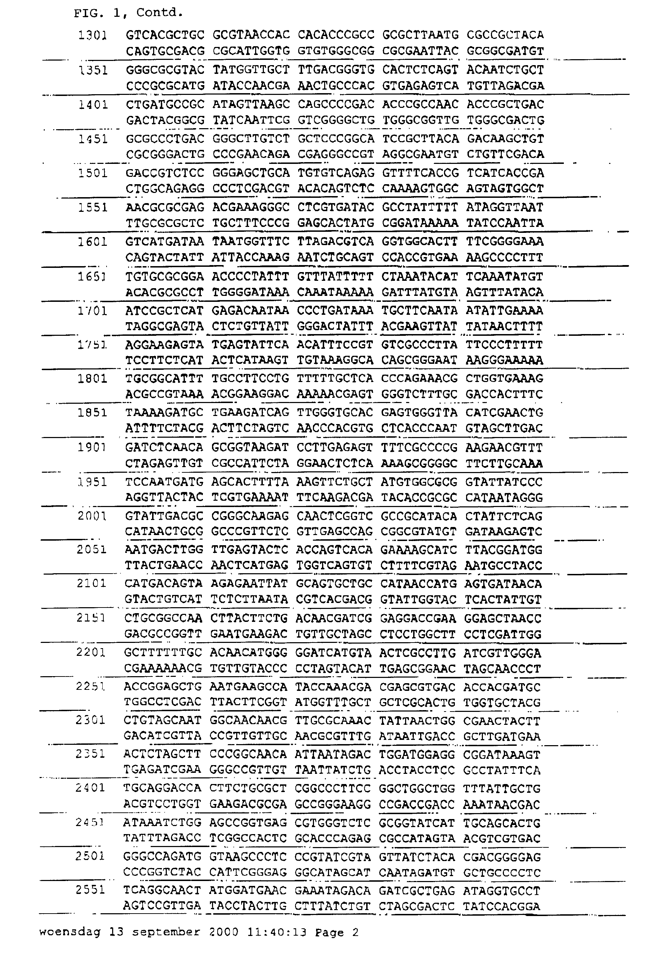

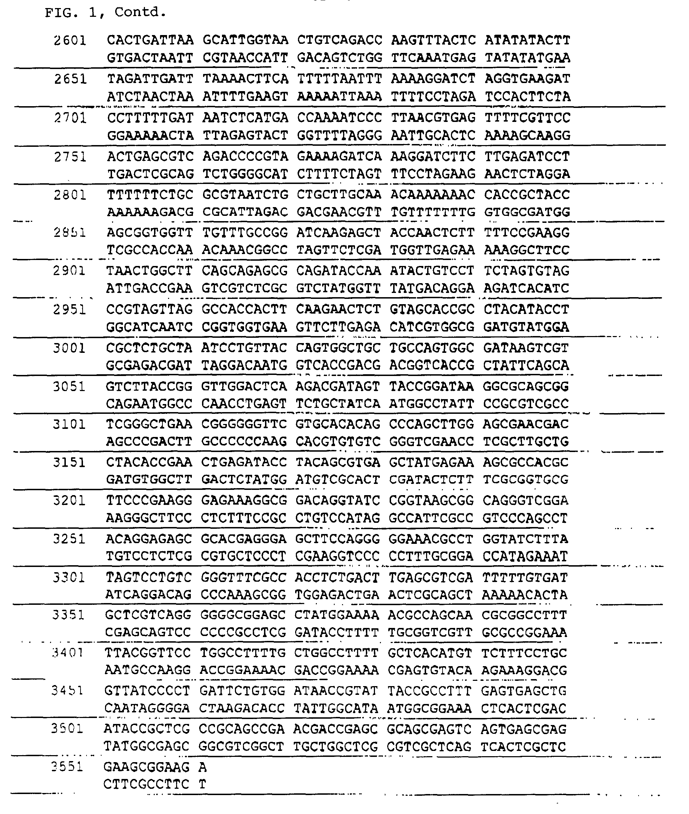

The phagemid PDV U03 is the basis vector for generating a library of binding molecules consisting of variable heavy chain 3 domains. A nucleic acid sequence of the phagemid PDV U03 is given in figure 1. Instead of gVIIIp protein in the PDV UO3 vector gIIIp can also be used.

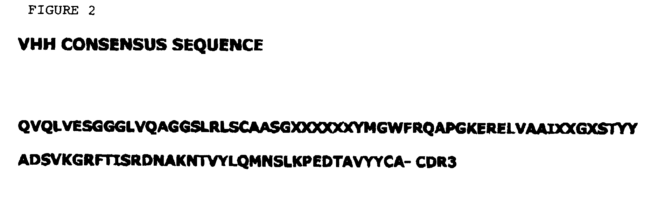

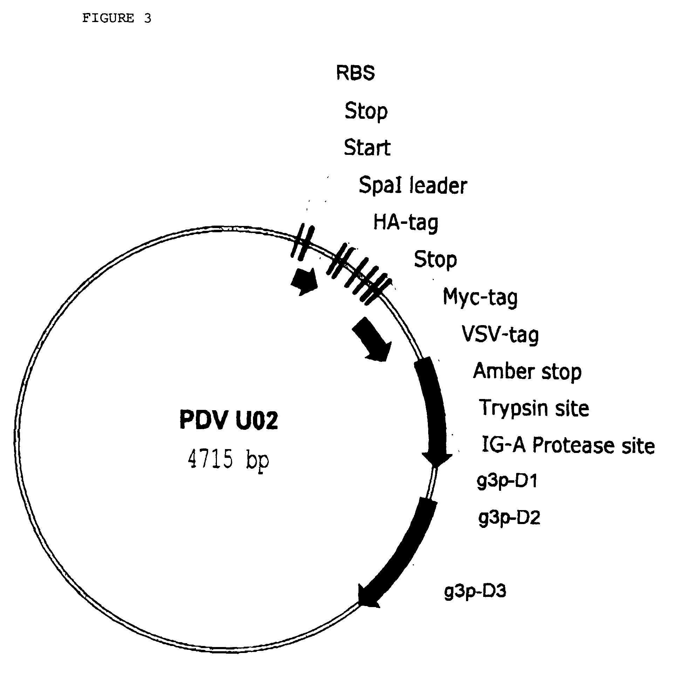

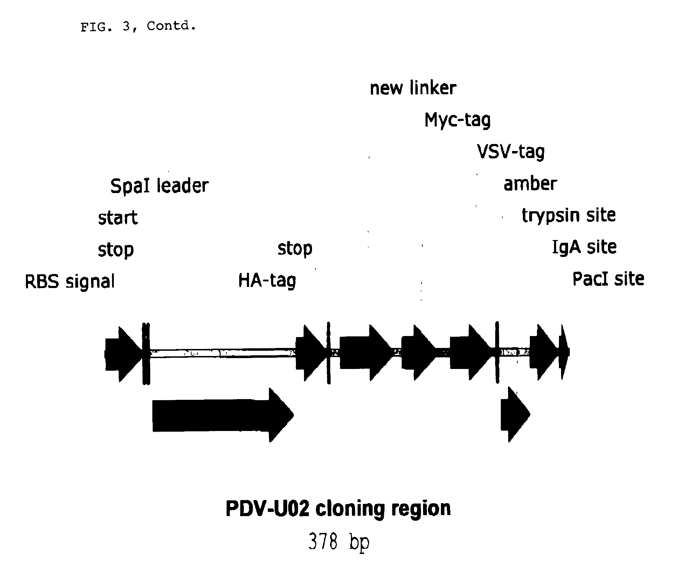

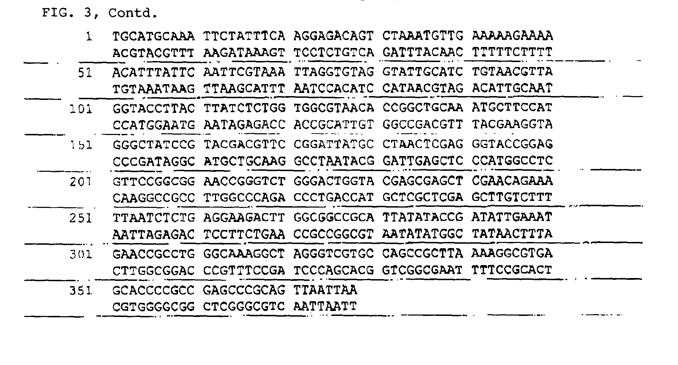

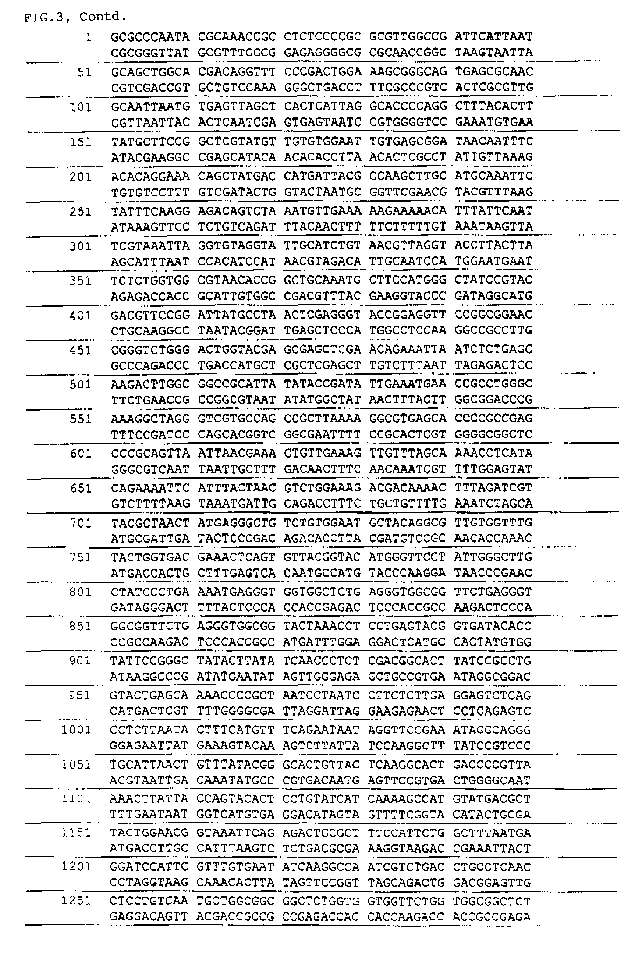

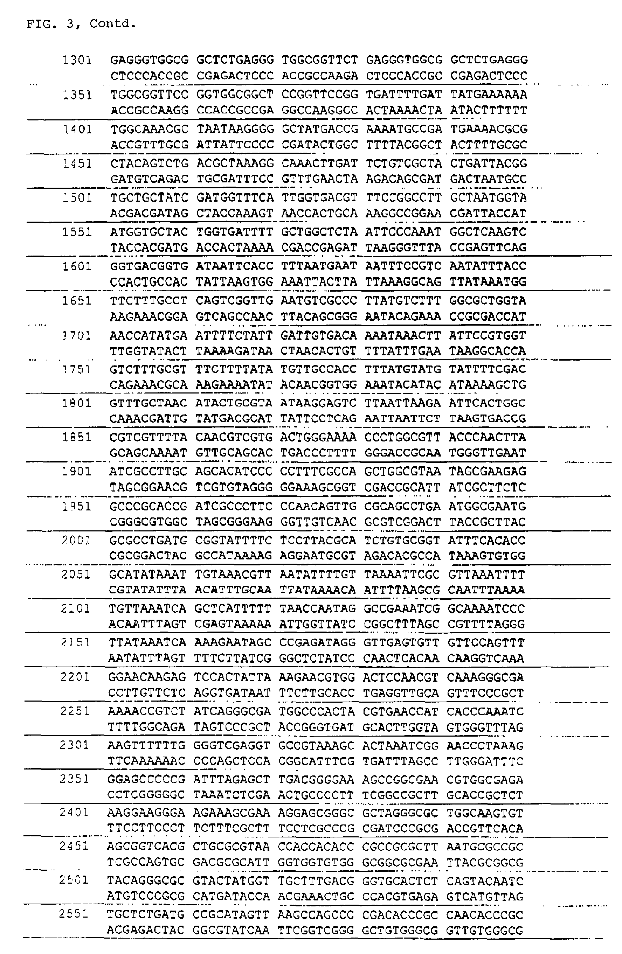

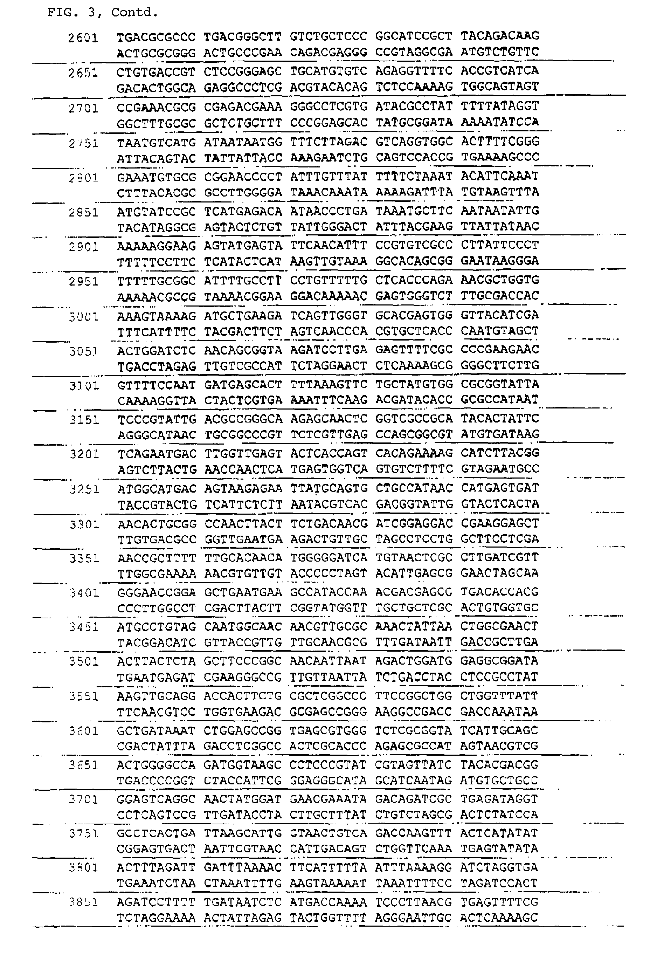

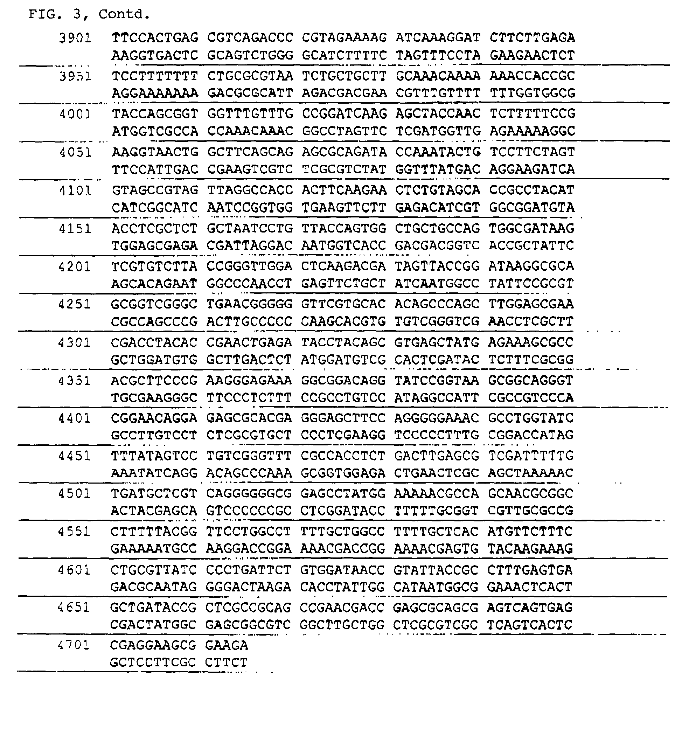

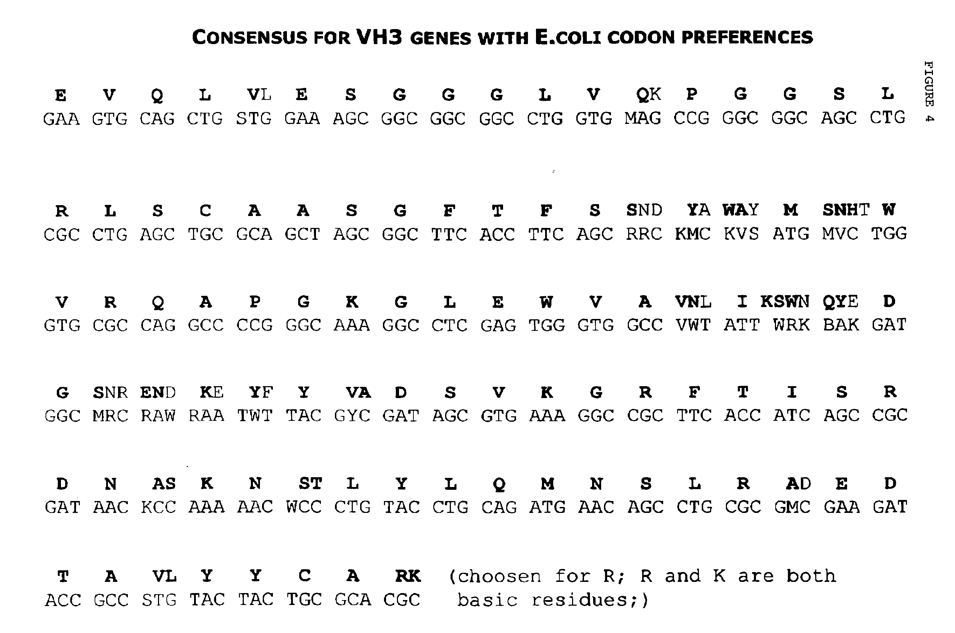

The core of the soluble VH3 domain is given in figure 2. The dots indicate places, representing CDR1 and CDR2 in an unaltered VH domain, where through varying the amino acid sequence, VH domains of various binding specificities can be obtained. The place marked "CDR3" in the figure, also indicates a place where through varying amino acids, VH domains comprising various binding specificities can be obtained. Of course said CDR3 regions may vary in size, at least according to the natural VH3 size variation in CDR3. By varying the amino acid sequence in the CDR regions it is possible to generate VH3 domains with varying specificities. The solubility of sVH3 versus an unmodified VH3 is due to mutations in framework 2 and framework 3, said mutations leading to a change in the hydrophobicity of the VH3 domain such that the hydrophilicity of the mutated VH3 domain increases. The solubility of sVH3 allows the generation of a phage comprising a binding molecule consisting of a VH domain in the absence of a light chain.

Libraries of binding specificities based on sVH3 domains can be generated by methods known in the art as long as the basic amino-acid sequence given in figure 2 is used. Other amino-acid sequences then given in figure 2 can also be used provided that they result in a sufficiently soluble VH3 domain. A person skilled in the art can arrive at the library by for instance chosen primers with at least partial overlap and building an ever larger part of the sVH3 domain by consecutively amplifying resulting product with a further partially overlapping primer. The CDR3 domain being located at the extreme end of the VH domain requires attention in the amplification procedure. Preferably, one or more (partially overlapping) primers are used that result in a restriction site being present at the extreme end of the amplified product such that the resulting sVH3 library can easily be cloned into PDV UO3. A preferred combination of enzymes to clone the library into PDV UO3 is NcoI and XhoI, wherein NcoI is located near the leader in PDV UO3 that is fused to the start of the sVH3 domain.

The resulting phagemids are electroporated into E. coli TG1 or XL1-blueTEN. The bacteria are plated onto suitable culture plates that include 5% glucose. The next day the resulting colonies are collected and stored. Several of these collections are inoculated in liquid medium and helper phages. After 1 night at degrees 30 C the phages are harvested. The resulting phages are selected for the appropriate target and amplified using said bacteria. The amplified phages were sequenced and shown to be as expected.

Generation of a structural protein capable of supporting proper VH3 function.

The phagemid PDV UO2 is the basis vector for generating a library of binding molecules consisting of variable heavy chain 3 domains further comprising a structural protein (SP) capable of supporting VH3 function. (SP does not comprise intrinsic antigen binding capacity). The sequence of a first SP (SP1) is obtained by shortening the binding loops of CDR1 and CDR2 in the light chain Vκ3 such that the binding properties are destroyed but the heavy chain supporting function of the light chain is essentially left intact. This is achieved by deleting amino acid from CDR1 and CDR2 such that these CDRs do not contain antigen binding capacity. In this Example, the 4 amino acids representing amino acid 28-31 are omitted from CDR1. These amino acids represent the most variable region in the CDR1 region of Vκ1 (O12). From CDR2, 3 amino acids, representing amino acid 53-55 in Vκ1 (O12) are omitted. Vκ1 CDR3 is replaced by a VSV-tag. The VSV-tag used contains the amino acid sequence YTDIEMNRLGK. A nucleic acid encoding SP1 was generated synthetically using assembly PCR and the correctness of the nucleic acid sequence was verified by sequencing. The nucleic acid contains a *Not*I site and a *Sac*I site such that cloning of SP1 into PDV UO2 does not disrupt the reading frame of the gIII protein. The *Not*I site is located near the putative N-terminal part of SP1.

SP2 was generated based on Vκ3 (A27) by omitting the 5 amino acids representing amino acid 28-31A are omitted from CDR1. These amino acids represent the most variable region in the CDR1 region of Vκ3 (A27). From CDR2, 3 amino acids, representing amino acid 53-55 in Vκ3 (A27) are omitted. The CDR3 of Vκ3 (A27) is replaced by a VSV-tag. The VSV-tag used contains the amino acid sequence YTDIEMNRLGK. A nucleic acid encoding SP2 was generated synthetically using assembly PCR and the correctness of the nucleic acid sequence was verified by sequencing. The nucleic acid contains a *Not*I site and a *Sac*I site such that cloning of SP2 into PDV U02 does not disrupt the reading frame of the gIII protein. The *Not*I site is located near the putative N-terminal part of SP2.

A VH3 framework and CDR1 and CDR2 randomized region used in this example is depicted in figure 4. The nucleic acid sequence encoding this VH3 framework is also given in figure 4. This nucleic sequence is optimized for codon usage in both *E. coli* and human cells. Table I depicts nucleic acid sequences that are optimized for codon usage in *E. coli* and human cells. The nucleic acid sequences encoding the framework are flanked by restriction sites *Nco*I and *Xho*I such that the reading frame of the gIII protein is left intact. The framework is cloned into PDV UO2 using the sites indicated.

The resulting phagemids containing either SP1 together with the framework or SP2 together with the frame work are electroporated into *E. coli* TG1 or XL1-blueTEN. The bacteria are plated onto suitable culture plates that include 5% glucose. The next day the resulting colonies are collected and stored. Several of these collections are inoculated in liquid medium and helper phages. After 1 night at 30 degrees C, the phages are harvested. The resulting phages are selected for the appropriate target and amplified using said bacteria. The amplified phages were sequenced and shown to be as expected.

### References

1 Berek, C., & Milstein, C. 1987. Mutation drift and repertoire shift in the maturation of the immune response. Immunol. Rev. 96:23.
2 Winter, G. & Milstein, C. 1991. Man-made antibodies. Nature. 349:293.
3 Vaughan, T.J., Osbourn, J.K., & Tempest, P.R. 1998. Human antibodies by design. Nat. Biotechnol. 16,535.
4 Winter, G., Griffiths, A.D., Hawkins, R.E., & Hoogenboom, H.R. 1994. Making antibodies by phage display technology. Annu. Rev. Immunol. 12:433.
5 Burton, D.R., & Barbas, C.F. 1994. Human antibodies from combinatorial libraries. Adv.Immunol. 57:191.
6 Hoogenboom, H.R. 1994. Designing and optimizing library selection strategies for generating high-affinity antibodies. Trends in Biotechnol. 15:62.
7 Marks, J.D., Griffiths, A.D., Malmqvist, M., Clackson, T., Bye, J.M., & Winter, G. 1992. Bypassing immunisation: high affinity human antibodies by chain shuffling. Bio/Technology. 10:779.
8 Clackson, T., Hoogenboom, H.R., Griffiths, A.D., & Winter, G. 1991. Making antibody fragments using phage display libraries. Nature. 352:624.
9 Hawkins, R.E., Russel, S.J., & Winter.G. 1992. Selection of phage antibodies by binding affinity: mimicking affinity maturation. J.Mol.Biol. 226:889.
10 Low, N.M., Holliger, P.H., & Winter, G. 1996. Mimicking somatic hypermutation: affinity maturation of antibodies displayed on bacteriophage using a bacterial mutator strain. J.Mol.Biol. 260,359.
11 Barbas, C.F., Hu, D., Dunlop, N., Sawyer, L., Cababa, D., Hendry, R.M., Nara, P.L., & Burton, D.R. 1994. In vitro evolution of a neutralizing human antibody to human immunodeficiency virus type 1 to enhance affinity and broaden strain cross-reactivity. Proc. Natl. Acad. Sci. USA. 91:3809.
12 Yang, W.-P., Green, K., Pinz-Sweeney, S., Briones, A.T., Burton, D.R., & Barbas, C.F. 1995. CDR walking mutagenesis for the affinity maturation of a potent human ant-HIV-1 antibody into the picomolar range. J. Mol. Biol. 254:392.
13Balint, R.F., & Larrick, J.W. 1993. Antibody engineering by parsimonious mutagenesis. Gene. 137:109.

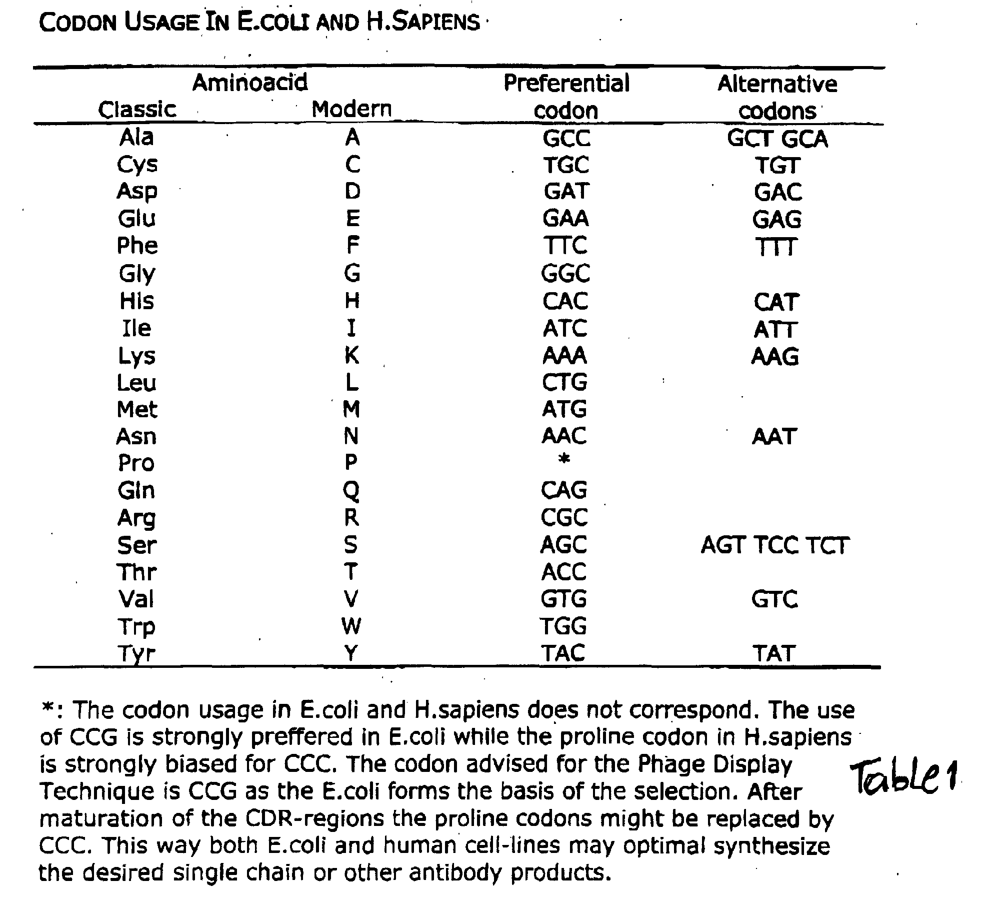

## Claims

1. A method for producing a human monoclonal antibody, said method comprising:
providing a library of binding molecules, the binding domain of which consists essentially of human heavy chain variable fragments in a functional format,
selecting from said library at least one heavy chain variable fragment having a desired binding affinity, and
inserting a nucleic acid encoding said heavy chain variable fragment into a nucleic acid encoding the complementary part of at least a heavy chain of said human monoclonal antibody, allowing for expression of the resulting heavy chain and for assembly of said heavy chain with a desired light chain, and producing a human monoclonal antibody.

2. The method according to claim 1 wherein said heavy chain variable fragment is in a functional format through fusion to a structural protein designed for that purpose.

3. The method according to claim 1, wherein at least one sequence of said heavy chain variable fragment relevant only for association with a light chain is removed.

4. The method according to claim 1, wherein the complementary part of the heavy chain is derived from VH3, VH4 or VH1.

5. The method according to claim 1, wherein the light chain is derived from to a member of a Vkappa1, Vkappa3 and Vlambda3 gene family.

6. A human monoclonal antibody obtainable by a method according to claims 1-5.

7. A method for producing a structural amino acid sequence or a nucleic acid sequence encoding such an amino acid sequence for keeping a human heavy chain variable fragment in a functional format upon expression of a nucleic acid encoding such a fragment in a fusion with a nucleic acid encoding a protein expressed associated with the surface of a phage particle, said method comprising:
fusing a nucleic acid sequence encoding a possible structural amino acid sequence to a nucleic acid which is a fusion of a human heavy chain variable fragment with a known binding affinity and said nucleic acid encoding a protein expressed associated with the surface of a phage particle, and
expressing said nucleic acid in the context of a suitable phage expression system and selecting fusions which expose the desired binding affinity.

8. A proteinaceous substance or a nucleic acid encoding it, which substance is capable of keeping a heavy chain variable fragment in a functional conformation, obtainable by a method according to claim 7.

9. A method for making a library for use in a method according to claim 1, said method comprising:
cloning a number of randomized nucleic acids derived from a heavy chain variable fragment in functional alignment with a nucleic acid encoding a proteinaceous substance according to claim 8, and
providing the resulting nucleic acid in functional alignment with a nucleic acid encoding a protein expressed associated with the surface of a phage particle and expressing the resulting nucleic acids comprising said heavy chain variable fragment, said proteinaceous substance encoding acid and said surface protein encoding nucleic acid in the context of a suitable phage expression system, thus producing said library.

10. A phage display library obtainable by a method according to claim 9.


### Annex to the application documents - subsequently filed sequences listing

SEQUENCE LISTING

<110> U-BiSys B.V.

<120> Heavy chain libraries

<130> P54234EP00

<140> 00203216.7
<141> 2000-09-15

<160> 7

<170> PatentIn Ver. 2.1

<210> 1
<211> 11
<212> PRT
<213> Artificial Sequence

<220>
<223> Description of Artificial Sequence: VSV-tag

<220>
<221> SITE
<222> (1)..(11)

<400> 1
Tyr Thr Asp Ile Glu Met Asn Arg Leu Gly Lys
  1               5                   10

<210> 2
<211> 3561
<212> DNA
<213> Artificial Sequence

<220>
<223> Description of Artificial Sequence: Phagemid PDV UO3

<220>
<221> misc_feature
<222> (1)..(3561)

<400> 2
gcgcccaata cgcaaaccgc ctctccccgc gcgttggccg attcattaat gcagctggca 60

cgacaggttt cccgactgga aagcgggcag tgagcgcaac gcaattaatg tgagttagct 120

cactcattag gcaccccagg ctttacactt tatgcttccg gctcgtatgt tgtgtggaat 180

tgtgagcgga taacaatttc acacaggaaa cagctatgac catgattacg ccaagcttgc 240

atgcaaattc tatttcaagg agacagtcta aatgttgaaa aagaaaaaca tttattcaat 300

tcgtaaatta ggtgtaggta ttgcatctgt aacgttaggt accttactta tctctggtgg 360

cgtaacaccg gctgcaaatg cttccatggg ctatccgtac gacgttccgg attatgccta 420

actcgagtta tataccgata ttgaaatgaa ccgcctgggc aaaggcggtc gtgccagccg 480

cttaaaaggc gtgagcaccc cgccgagccc gcagttaatt aacgctgagg gtgacgatcc 540

cgcaaaagcg gcctttgact ccctgcaagc ctcagcgacc gaatatatcg gttatgcgtg 600

ggcgatggtt gttgtcattg tcggcgcaac tatcggtatc aagctgttta agaaattcac 660

ctcgaaagca agctgattaa ttaagaattc actggccgtc gttttacaac gtcgtgactg 720

ggaaaaccct ggcgttaccc aacttaatcg ccttgcagca catccccctt tcgccagctg 780

gcgtaatagc gaagaggccc gcaccgatcg cccttcccaa cagttgcgca gcctgaatgg 840

cgaatggcgc ctgatgcggt attttctcct tacgcatctg tgcggtattt cacaccgcat 900

ataaattgta aacgttaata ttttgttaaa attcgcgtta aatttttgtt aaatcagctc 960

attttttaac caataggccg aaatcggcaa aatcccttat aaatcaaaag aatagcccga 102
0
gatagggttg agtgttgttc cagtttggaa caagagtcca ctattaaaga acgtggactc 108
0
caacgtcaaa gggcgaaaaa ccgtctatca gggcgatggc ccactacgtg aaccatcacc 114
0
caaatcaagt tttttggggt cgaggtgccg taaagcacta aatcggaacc ctaaagggag 120
0
cccccgattt agagcttgac ggggaaagcc ggcgaacgtg gcgagaaagg aagggaagaa 126
0
agcgaaagga gcgggcgcta gggcgctggc aagtgtagcg gtcacgctgc gcgtaaccac 132
0
cacacccgcc gcgcttaatg cgccgctaca gggcgcgtac tatggttgct ttgacgggtg 138
0
cactctcagt acaatctgct ctgatgccgc atagttaagc cagccccgac acccgccaac 144
0
acccgctgac gcgccctgac gggcttgtct gctcccggca tccgcttaca gacaagctgt 150
0
gaccgtctcc gggagctgca tgtgtcagag gttttcaccg tcatcaccga aacgcgcgag 156
0
acgaaagggc ctcgtgatac gcctattttt ataggttaat gtcatgataa taatggtttc 162
0
ttagacgtca ggtggcactt ttcggggaaa tgtgcgcgga acccctattt gtttattttt 168
0
ctaaatacat tcaaatatgt atccgctcat gagacaataa ccctgataaa tgcttcaata 174
0
atattgaaaa aggaagagta tgagtattca acatttccgt gtcgccctta ttcccttttt 180
0
tgcggcattt tgccttcctg tttttgctca cccagaaacg ctggtgaaag taaaagatgc 186
0
tgaagatcag ttgggtgcac gagtgggtta catcgaactg gatctcaaca gcggtaagat 192
0
ccttgagagt tttcgccccg aagaacgttt tccaatgatg agcactttta aagttctgct 198

0
atgtggcgcg gtattatccc gtattgacgc cgggcaagag caactcggtc gccgcataca 204
0
ctattctcag aatgacttgg ttgagtactc accagtcaca gaaaagcatc ttacggatgg 210
0
catgacagta agagaattat gcagtgctgc cataaccatg agtgataaca ctgcggccaa 216
0
cttacttctg acaacgatcg gaggaccgaa ggagctaacc gcttttttgc acaacatggg 222
0
ggatcatgta actcgccttg atcgttggga accggagctg aatgaagcca taccaaacga 228
0
cgagcgtgac accacgatgc ctgtagcaat ggcaacaacg ttgcgcaaac tattaactgg 234
0
cgaactactt actctagctt cccggcaaca attaatagac tggatggagg cggataaagt 240
0
tgcaggacca cttctgcgct cggcccttcc ggctggctgg tttattgctg ataaatctgg 246
0
agccggtgag cgtgggtctc gcggtatcat tgcagcactg gggccagatg gtaagccctc 252
0
ccgtatcgta gttatctaca cgacggggag tcaggcaact atggatgaac gaaatagaca 258
0
gatcgctgag ataggtgcct cactgattaa gcattggtaa ctgtcagacc aagtttactc 264
0
atatatactt tagattgatt taaaacttca tttttaattt aaaaggatct aggtgaagat 270
0
cctttttgat aatctcatga ccaaaatccc ttaacgtgag ttttcgttcc actgagcgtc 276
0
agaccccgta gaaaagatca aaggatcttc ttgagatcct ttttttctgc gcgtaatctg 282
0
ctgcttgcaa acaaaaaaac caccgctacc agcggtggtt tgtttgccgg atcaagagct 288
0
accaactctt tttccgaagg taactggctt cagcagagcg cagataccaa atactgtcct 294
0
tctagtgtag ccgtagttag gccaccactt caagaactct gtagcaccgc ctacatacct 300
0
cgctctgcta atcctgttac cagtggctgc tgccagtggc gataagtcgt gtcttaccgg 306
0
gttggactca agacgatagt taccggataa ggcgcagcgg tcgggctgaa cggggggttc 312
0
gtgcacacag cccagcttgg agcgaacgac ctacaccgaa ctgagatacc tacagcgtga 318
0
gctatgagaa agcgccacgc ttcccgaagg gagaaaggcg gacaggtatc cggtaagcgg 324
0
cagggtcgga acaggagagc gcacgaggga gcttccaggg ggaaacgcct ggtatcttta 330
0
tagtcctgtc gggtttcgcc acctctgact tgagcgtcga tttttgtgat gctcgtcagg 336
0
ggggcggagc ctatggaaaa acgccagcaa cgcggccttt ttacggttcc tggccttttg 342
0
ctggcctttt gctcacatgt tctttcctgc gttatcccct gattctgtgg ataaccgtat 348
0
taccgccttt gagtgagctg ataccgctcg ccgcagccga acgaccgagc gcagcgagtc 354
0
agtgagcgag gaagcggaag a 356
1

<210> 3
<211> 96
<212> PRT
<213> Artificial Sequence

<220>
<223> Description of Artificial Sequence: Soluble VH3 domain core

<220>
<221> SITE
<222> (1)..(96)

<400> 3
Gln Val Gln Leu Val Glu Ser Gly Gly Gly Leu Val Gln Ala Gly Gly
1               5                   10                  15

Ser Leu Arg Leu Ser Cys Ala Ala Ser Gly Xaa Xaa Xaa Xaa Xaa Xaa
            20                  25                  30

Tyr Met Gly Trp Phe Arg Gln Ala Pro Gly Lys Glu Arg Glu Leu Val
        35                  40                  45

Ala Ala Ile Xaa Xaa Gly Xaa Ser Thr Tyr Tyr Ala Asp Ser Val Lys
    50                  55                  60

Gly Arg Phe Thr Ile Ser Arg Asp Asn Ala Lys Asn Thr Val Tyr Leu
65                  70                  75                  80

Gln Met Asn Ser Leu Lys Pro Glu Asp Thr Ala Val Tyr Tyr Cys Ala
                85                  90                  95

<210> 4
<211> 378
<212> DNA
<213> Artificial Sequence

<220>
<223> Description of Artificial Sequence: PDV-UO2 cloning region

<220>
<221> misc_feature
<222> (1)..(378)

<400> 4
tgcatgcaaa ttctatttca aggagacagt ctaaatgttg aaaaagaaaa acatttattc 60

aattcgtaaa ttaggtgtag gtattgcatc tgtaacgtta ggtaccttac ttatctctgg 120

tggcgtaaca ccggctgcaa atgcttccat gggctatccg tacgacgttc cggattatgc 180

ctaactcgag ggtaccggag gttccggcgg aaccgggtct gggactggta cgagcgagct 240

cgaacagaaa ttaatctctg aggaagactt ggcggccgca ttatataccg atattgaaat 300

gaaccgcctg ggcaaaggct agggtcgtgc cagccgctta aaaggcgtga gcaccccgcc 360

gagcccgcag ttaattaa 378

<210> 5
<211> 4715
<212> DNA
<213> Artificial Sequence

<220>
<223> Description of Artificial Sequence: PDV-UO2 sequence

<220>
<221> misc_feature
<222> (1)..(4715)

<400> 5
gcgcccaata cgcaaaccgc ctctccccgc gcgttggccg attcattaat gcagctggca 60

cgacaggttt cccgactgga aagcgggcag tgagcgcaac gcaattaatg tgagttagct 120

cactcattag gcaccccagg ctttacactt tatgcttccg gctcgtatgt tgtgtggaat 180

tgtgagcgga taacaatttc acacaggaaa cagctatgac catgattacg ccaagcttgc 240

atgcaaattc tatttcaagg agacagtcta aatgttgaaa aagaaaaaca tttattcaat 300

tcgtaaatta ggtgtaggta ttgcatctgt aacgttaggt accttactta tctctggtgg 360

cgtaacaccg gctgcaaatg cttccatggg ctatccgtac gacgttccgg attatgccta 420

actcgagggt accggaggtt ccggcggaac cgggtctggg actggtacga gcgagctcga 480

acagaaatta atctctgagc aagacttggc ggccgcatta tataccgata ttgaaatgaa 540

ccgcctgggc aaaggctagg gtcgtgccag ccgcttaaaa ggcgtgagca ccccgccgag 600

cccgcagtta attaacgaaa ctgttgaaag ttgtttagca aaacctcata cagaaaattc 660

atttactaac gtctggaaag acgacaaaac tttagatcgt tacgctaact atgagggctg 720

tctgtggaat gctacaggcg ttgtggtttg tactggtgac gaaactcagt gttacggtac 780

atgggttcct attgggcttg ctatccctga aaatgagggt ggtggctctg agggtggcgg 840

ttctgagggt ggcggttctg agggtggcgg tactaaacct cctgagtacg gtgatacacc 900

tattccgggc tatacttata tcaaccctct cgacggcact tatccgcctg gtactgagca 960

aaaccccgct aatcctaatc cttctcttga ggagtctcag cctcttaata ctttcatgtt 102
0
tcagaataat aggttccgaa ataggcaggg tgcattaact gtttatacgg gcactgttac 108
0

tcaaggcact gaccccgtta aaacttatta ccagtacact cctgtatcat caaaagccat 114
0
gtatgacgct tactggaacg gtaaattcag agactgcgct ttccattctg gctttaatga 120
0
ggatccattc gtttgtgaat atcaaggcca atcgtctgac ctgcctcaac ctcctgtcaa 126
0
tgctggcggc ggctctggtg gtggttctgg tggcggctct gagggtggcg gctctgaggg 132
0
tggcggttct gagggtggcg gctctgaggg tggcggttcc ggtggcggct ccggttccgg 138
0
tgattttgat tatgaaaaaa tggcaaacgc taataagggg gctatgaccg aaaatgccga 144
0
tgaaaacgcg ctacagtctg acgctaaagg caaacttgat tctgtcgcta ctgattacgg 150
0
tgctgctatc gatggtttca ttggtgacgt ttccggcctt gctaatggta atggtgctac 156
0
tggtgatttt gctggctcta attcccaaat ggctcaagtc ggtgacggtg ataattcacc 162
0
tttaatgaat aatttccgtc aatatttacc ttctttgcct cagtcggttg aatgtcgccc 168
0
ttatgtcttt ggcgctggta aaccatatga attttctatt gattgtgaca aaataaactt 174
0
attccgtggt gtctttgcgt ttcttttata tgttgccacc tttatgtatg tattttcgac 180
0
gtttgctaac atactgcgta ataaggagtc ttaattaaga attcactggc cgtcgtttta 186
0
caacgtcgtg actgggaaaa ccctggcgtt acccaactta atcgccttgc agcacatccc 192
0
cctttcgcca gctggcgtaa tagcgaagag gcccgcaccg atcgcccttc ccaacagttg 198
0
cgcagcctga atggcgaatg gcgcctgatg cggtattttc tccttacgca tctgtgcggt 204
0
atttcacacc gcatataaat tgtaaacgtt aatattttgt taaaattcgc gttaaatttt 210
0
tgttaaatca gctcattttt taaccaatag gccgaaatcg gcaaaatccc ttataaatca 216
0
aaagaatagc ccgagatagg gttgagtgtt gttccagttt ggaacaagag tccactatta 222
0
aagaacgtgg actccaacgt caaagggcga aaaaccgtct atcagggcga tggcccacta 228
0
cgtgaaccat cacccaaatc aagttttttg gggtcgaggt gccgtaaagc actaaatcgg 234
0
aaccctaaag ggagcccccg atttagagct tgacggggaa agccggcgaa cgtggcgaga 240
0
aaggaaggga agaaagcgaa aggagcgggc gctagggcgc tggcaagtgt agcggtcacg 246
0
ctgcgcgtaa ccaccacacc cgccgcgctt aatgcgccgc tacagggcgc gtactatggt 252
0
tgctttgacg ggtgcactct cagtacaatc tgctctgatg ccgcatagtt aagccagccc 258
0
cgacacccgc caacacccgc tgacgcgccc tgacgggctt gtctgctccc ggcatccgct 264
0
tacagacaag ctgtgaccgt ctccgggagc tgcatgtgtc agaggttttc accgtcatca 270
0
ccgaaacgcg cgagacgaaa gggcctcgtg atacgcctat ttttataggt taatgtcatg 276
0

ataataatgg tttcttagac gtcaggtggc acttttcggg gaaatgtgcg cggaacccct 282
0
atttgtttat ttttctaaat acattcaaat atgtatccgc tcatgagaca ataaccctga 288
0
taaatgcttc aataatattg aaaaaggaag agtatgagta ttcaacattt ccgtgtcgcc 294
0
cttattccct tttttgcggc attttgcctt cctgtttttg ctcacccaga aacgctggtg 300
0
aaagtaaaag atgctgaaga tcagttgggt gcacgagtgg gttacatcga actggatctc 306
0
aacagcggta agatccttga gagttttcgc cccgaagaac gttttccaat gatgagcact 312
0
tttaaagttc tgctatgtgg cgcggtatta tcccgtattg acgccgggca agagcaactc 318
0
ggtcgccgca tacactattc tcagaatgac ttggttgagt actcaccagt cacagaaaag 324
0
catcttacgg atggcatgac agtaagagaa ttatgcagtg ctgccataac catgagtgat 330
0
aacactgcgg ccaacttact tctgacaacg atcggaggac cgaaggagct aaccgctttt 336
0
ttgcacaaca tgggggatca tgtaactcgc cttgatcgtt gggaaccgga gctgaatgaa 342
0
gccataccaa acgacgagcg tgacaccacg atgcctgtag caatggcaac aacgttgcgc 348
0
aaactattaa ctggcgaact acttactcta gcttcccggc aacaattaat agactggatg 354
0
gaggcggata aagttgcagg accacttctg cgctcggccc ttccggctgg ctggtttatt 360
0
gctgataaat ctggagccgg tgagcgtggg tctcgcggta tcattgcagc actggggcca 366
0
gatggtaagc cctcccgtat cgtagttatc tacacgacgg ggagtcaggc aactatggat 372
0
gaacgaaata gacagatcgc tgagataggt gcctcactga ttaagcattg gtaactgtca 378
0
gaccaagttt actcatatat actttagatt gatttaaaac ttcattttta atttaaaagg 384
0
atctaggtga agatcctttt tgataatctc atgaccaaaa tcccttaacg tgagttttcg 390
0
ttccactgag cgtcagaccc cgtagaaaag atcaaaggat cttcttgaga tccttttttt 396
0
ctgcgcgtaa tctgctgctt gcaaacaaaa aaaccaccgc taccagcggt ggtttgtttg 402
0
ccggatcaag agctaccaac tctttttccg aaggtaactg gcttcagcag agcgcagata 408
0
ccaaatactg tccttctagt gtagccgtag ttaggccacc acttcaagaa ctctgtagca 414
0
ccgcctacat acctcgctct gctaatcctg ttaccagtgg ctgctgccag tggcgataag 420
0
tcgtgtctta ccgggttgga ctcaagacga tagttaccgg ataaggcgca gcggtcgggc 426
0
tgaacggggg gttcgtgcac acagcccagc ttggagcgaa cgacctacac cgaactgaga 432
0
tacctacagc gtgagctatg agaaagcgcc acgcttcccg aagggagaaa ggcggacagg 438
0
tatccggtaa gcggcagggt cggaacagga gagcgcacga gggagcttcc agggggaaac 444
0

gcctggtatc tttatagtcc tgtcgggttt cgccacctct gacttgagcg tcgatttttg 450
0
tgatgctcgt caggggggcg gagcctatgg aaaaacgcca gcaacgcggc ctttttacgg 456
0
ttcctggcct tttgctggcc ttttgctcac atgttctttc ctgcgttatc ccctgattct 462
0
gtggataacc gtattaccgc ctttgagtga gctgataccg ctcgccgcag ccgaacgacc 468
0
gagcgcagcg agtcagtgag cgaggaagcg gaaga 471
5

<210> 6
<211> 98
<212> PRT
<213> Artificial Sequence

<220>
<223> Description of Artificial Sequence: Part of variable fragment

<220>
<221> SITE
<222> (1)..(98)
<223> /note="Position 5 can also be L, position 13 can also be K, pos
ition 31
can also be N/D, position 32 can also be A, position 33 can al
so be A/Y,
position 35 can also be N/H/T, position 50 can also be N/L, po
sition
52 can also be S/W/N, position 53 can also be Y/E, position 56
can also
be N/R, position 57 can also be N/D, position 58 can also be E
, position
59 can also be F, position 61 can also be A, position 75 can a
lso be S,
position 78 can also be T, position 88 can also be D, position
93 can
also be L, position 98 can also be K.

<400> 6
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Trp Met Ser Trp Val Arg Gln Ala Pro Gly Lys Gly Leu Glu Trp Val
        35                  40                  45

Ala Val Ile Lys Gln Asp Gly Ser Glu Lys Tyr Tyr Val Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys

                85                  90                  95

Ala Arg

<210> 7
<211> 294
<212> DNA
<213> Artificial Sequence

<220>
<223> Description of Artificial Sequence: Nucleic Acid sequence encod
ing

<220>
<221> misc_feature
<222> (1)..(294)

<400> 7
gaagtgcagc tgstggaaag cggcggcggc ctggtgmagc cgggcggcag cctgcgcctg 60

agctgcgcag ctagcggctt caccttcagc rrckmckvsa tgmvctgggt gcgccaggcc 120

ccgggcaaag gcctcgagtg ggtggccvwt attwrkbakg atggcmrcra wraatwttac 180

gycgatagcg tgaaaggccg cttcaccatc agccgcgata ackccaaaaa cwccctgtac 240

ctgcagatga acagcctgcg cgmcgaagat accgccstgt actactgcgc acgc 294